(19) [European Patent Office logo] Europäisches Patentamt / European Patent Office / Office européen des brevets

(11) **EP 1 938 096 B1**

(12) **EUROPEAN PATENT SPECIFICATION**

(45) Date of publication and mention
of the grant of the patent:
**30.12.2015 Bulletin 2015/53**

(21) Application number: **06799769.2**

(22) Date of filing: **17.10.2006**

(51) Int Cl.:
*G01N 33/497* [(2006.01)]     *B60K 28/06* [(2006.01)]

(86) International application number:
**PCT/SE2006/001169**

(87) International publication number:
**WO 2007/046745 (26.04.2007 Gazette 2007/17)**

(54) **A METHOD AND APPARATUS FOR ASSESSING BLOOD CONCENTRATION OF ETHANOL**

VERFAHREN UND VORRICHTUNG ZUR BEURTEILUNG DER BLUTKONZENTRATION VON ETHANOL

PROCEDE ET APPAREIL POUR EVALUER LA CONCENTRATION D'ETHANOL DANS LE SANG

(84) Designated Contracting States:
**AT BE BG CH CY CZ DE DK EE ES FI FR GB GR HU IE IS IT LI LT LU LV MC NL PL PT RO SE SI SK TR**

(30) Priority: **21.10.2005 GB 0521502**

(43) Date of publication of application:
**02.07.2008 Bulletin 2008/27**

(73) Proprietor: **Autoliv Development AB**
**447 83 Vårgårda (SE)**

(72) Inventors:
• **PETTERSSON, Håkan**
**S-448 34 Floda (SE)**

• **HÖK, Bertil**
**SE-723 44 Vasterås (SE)**
• **ANDERSSON, Gert**
**S-473 93 Lindome (SE)**

(74) Representative: **Beattie, Alex Thomas Stewart et al**
**Forresters**
**Skygarden**
**Erika-Mann-Strasse 11**
**80636 München (DE)**

(56) References cited:
**EP-A1- 1 441 212      EP-A2- 0 752 584**
**DE-A1- 4 235 328      DE-A1- 19 941 586**
**US-A- 3 505 022      US-A- 5 376 555**
**US-A1- 2003 136 600**

**Description**

THE PRESENT INVENTION relates to a method of assessing the blood concentration level, in a human or animal subject, of a volatile blood constituent, and also relates to an apparatus for implementing the method. Traffic injuries related to vehicle drivers influenced by alcohol or other drugs are a major problem in all modern societies. Many casualties and severe injuries can be attributed to this problem, associated with human tragedies of immense proportions, and huge economic losses. The search for preventive measures should be approached from all perspectives, leaving no possible solution untried. It is, however, also important that solutions are realistic from the points of view of human behaviour, reliability and economy.

The legislation concerning drugs and vehicle driving differs from one country to another, but generally reflects the severity of the problem in all modern societies. In Sweden and many other countries, vehicle driving is prohibited if the blood concentration of alcohol exceeds 0.02% (the exact number may vary from one country to another). The distinct concentration value, differentiating legal driving from illegal, calls for measurement routines that are not only reproducible but also legally unobjectable from all possible aspects of judicial security. In Sweden, direct blood sampling is compulsory for conviction of a person for drunk driving against his/her admission. Measurements by breath sampling, or through skin is thus considered less reliable.

Direct blood sampling is not practical for screening purposes or other applications on a large scale, due to cost, the problems of handling, and avoidance of infections. The transcutaneous approach is not likely to become successful for large-scale use for reasons of reliability. Breath sampling, on the other hand, is believed to have potential for development into useful large-scale solutions.

Various types of sensing devices capable of measuring the alcohol concentration within the expired air of a subject are already known. The gas exchange between the lung capillaries and the alveoli is normally highly efficient, thereby ensuring proper correlation between the measured gas concentration and the blood concentration.

Measurement of the concentration of alcohol or other volatile compounds in air can be performed with two distinctly different types of devices. In one type, classified as dispersive devices, the various constituents of the gas sample are dispersed according to a property which exhibits a significant and reproducible difference between each one of the constituents. Mass spectroscopy is one such method, relying on the fact that the molecular mass of each constituent exhibits such variation. Another method is gas chromatography, in which the dispersive property is the relative affinity of each constituent to a mobile gas carrier, and a stationary solid or liquid surface. Dispersive methods have common virtues of generality and precision, whereas they are relatively complex, and therefore expensive. In applications where the constituents are known, and the issue is to determine their relative concentrations, dispersive methods may be considered over-qualified.

Non-dispersive devices are commonly based on some property peculiar to the object of detection or measurement. Such a property could have a qualitative or quantitative nature. The latter means that the property as such is shared also by other substances, but may differ in magnitude, thereby enabling discrimination of the desirable substance from others. A qualitative nature means that the detection can be carried out regardless of quantity. From a basic perspective, the qualitative approach is favourable, since the risk of false detection is found to be reduced. In practice, however, the quantitative principle may offer other, and overruling advantages. Often, the final choice will depend on actual requirements of a specific application, and it is necessary to adapt the solution to such requirements.

Detection of alcohol is commonly performed by devices relying on the fact that alcohol is combustible, in contrast to the normal constituents of air. The combustion process takes place spontaneously at high temperatures, and at moderate temperatures in the presence of certain catalysts. The presence of alcohol, and its concentration, may be recorded by measuring the heat generated by the combustion, or by the consumption of oxygen. A sensor for this purpose could operate by the interaction between gas and solid elements, or could have a liquid interface. The amount of generated heat energy, or the quantity of oxygen consumed can be directly used for quantitative determination.

Common to all the devices mentioned above is that direct sampling is necessary. If the sample becomes diluted for one reason or another, the devices will record false value of too low a magnitude. Dilution may occur both by accident and deliberately. Leakage in any part of the sampling process may occur, and may in some cases be difficult both to avoid and detect. The risk for deliberate manipulation of the sampling process should be considered in view of the legal aspects of alcohol testing.

There is a close correlation between the blood concentration of a certain volatile constituent, and its concentration in the air expired by the subject being tested. This correlation relies upon the efficient equilibration of dissolved and free gas taking place in the alveoli/capillary interface within the lungs. The relation between dissolved and free gas is specific to a certain constituent, and is temperature dependent.

Ethyl alcohol, exhibiting a vapour pressure of 15.5 kPa at 37°C, is soluble in water. At equilibrium, the alcohol partial pressure above an alcohol/water solution will be directly related to the alcohol concentration in the solution. The corresponding relationship is valid for the air/blood interface. The efficiency of the equilibration process is dependent upon a large interface area, which is also crucial to the vital lung function within the subject. Disturbances, and thus limitations, of the assumed conditions for correlating expired air concentration with that in blood are thus closely related to healthy or diseased lung function. In subjects with lung disease, conclusions on the basis of measured concentrations within the expired air should be made very cautiously.

There are other problems related to the determination of the alcohol content of the expiratory airflow from a subject. When using conventional prior-proposed breath-testing apparatus, the subject is instructed to exhale into a mouthpiece connected to an alcohol sensor. In order to obtain adequate accuracy, the expired volume and flow must exceed certain limits to avoid dilution. For persons with lung disease, e g asthma, it may be difficult or even impossible to reach this limit. For hygienic reasons, mouthpieces are considered personal. Replacement of mouthpieces between different subjects obviously adds to the cost and time consumption of each measurement.

It is thought to be advantageous to provide a method which is contactless and which requires only a few seconds for completion.

EP-A-1441212 discloses a sensing device capable of detecting alcohol in the breath of a subject.

The present invention seeks to provide an improved method of assessing the blood concentration level, in a human or animal subject, of a volatile blood constituent, and an apparatus for the implementation of said method.

According to one aspect of the present invention, there is provided a method as defined in claim 1 hereinafter.

According to another aspect of the present invention, there is provided an apparatus as defined in claim 19 hereinafter.

So that the invention may be more readily understood, and so that further features thereof may be appreciated, embodiments of the invention will now be described, by way of example, with reference to the accompanying drawings, in which:

Figure 1 is a schematic flow chart illustrating the various steps of a method embodying the invention;

Figure 2 shows a number of plots, against time, for signals used in the method of the present invention;

Figure 3 is a schematic block diagram illustrating components of an apparatus in accordance with an embodiment of the present invention; and

Figure 4 shows several possible sensor elements suitable for use in the method and apparatus of the present invention.

The method of the present invention is based on the measurement of both alcohol-and carbon dioxide- concentrations in the expiratory air flowing from a subject's mouth, diluted in ambient air. As such, the method enables the use of a sensor arrangement which can be positioned at some distance from the mouth and nose of the subject, most preferably up to half a metre from the subject's mouth and nose. At such distances, the expired air flowing from the subject may be diluted as much as ten times with ambient air.

In accordance with the present invention, the concentrations of carbon dioxide and alcohol, $C_{extCO2}$ and $C_{extEtOH}$, are measured simultaneously at locations very close to one another within the expired air-flow from a subject such as a human. The degree of dilution in relation to corresponding alveolar concentrations is assumed to be equal for both gases, and comprised partly of the dead volume $V_{ds}$ in relation to the alveolar volume $V_{alv}$, and partly from the momentary dilution resulting from passage of the expired gas from the subject's nose and/or mouth to the location of the sensor arrangement. The dead volume $V_{ds}$ represents the volume of the subject's airways not participating in actual gas exchange. For an adult person, the dead volume is approximately 150ml which represents approximately 30% of the tidal volume (i.e. volume of air exchanged in each breath), and mainly consists of the upper airways.

The concentration of carbon dioxide and the concentration of alcohol, as measured within the expired air-flow can thus be defined by the following equations, where $C_{alvCO2}$ and $C_{alvEtOH}$ represent the alveolar carbon dioxide concentration and the alveolar alcohol concentration respectively....

$$C_{extCO2} = D \cdot \frac{V_{alv}}{V_{ds} + V_{alv}} \cdot C_{alvCO2} \qquad (1)$$

$$C_{extEtOH} = D \cdot \frac{V_{alv}}{V_{ds} + V_{alv}} \cdot C_{alvEtOH} \qquad (2)$$

By taking the ratio between (1) and (2), the dilution factors disappear, and the alveolar alcohol concentration can be determined from the following simple expression:

$$C_{alvEtOH} = \frac{C_{extEtOH}}{C_{extCO2}} \cdot C_{alvCO2} \qquad\qquad (3)$$

The alveolar or blood concentration of alcohol can thus be determined from the algorithm (3) basted on the externally measured ratio of signals representative of $C_{extEtOH}$ and $C_{extCO2}$, combined with an estimated or indirectly determined value representative of the subject's alveolar $CO_2$ concentration. In fact, the combined external/alveolar $CO_2$ measurement and/or estimation provides a representation of the degree of dilution of the expired airflow, allowing the alcohol signal to be related to that particular dilution and thus to represent the blood concentration. The representation is effectuated by the signal corresponding to external carbon dioxide, $C_{extCO2}$, and the estimation or indirect determination of alveolar carbon dioxide concentration, $C_{alvCO2}$. Alternatively, the external $CO_2$ measurement may be considered to represent a certain blood volume, enabling the alcohol signal to be related to that volume and to determination in absolute terms.

Algorithm (3) can also be used in applications where only discrete values of the signals representative of $C_{extEtOH}$ and $C_{extCO2}$ are of interest (for example to determine whether a certain threshold value of alcohol concentration has been exceeded or not, without the need to provide accurate instantaneous values for the detected concentration). In such a situation, the alcohol signal reading can be made at the instant that the $CO_2$ signal exceeds a certain predefined value.

The time and position dependence of the dilution factor D and the factor $V_{alv}/(V_{ds}+V_{alv})$ will require certain precautions, as will be outlined in more detail hereinafter. Another necessary condition for the method according to the invention is that adequate accuracy can be obtained for the external measurements, and the estimation or indirect measurement of alveolar (and arterial) $CO_2$ for the subject being tested.

By using presently available $CO_2$ sensors, based on infrared or electro-acoustic technology, a total measurement error of less than 2 percent for $C_{extCO2}$ has been found to be realistic, to a degree of dilution of ten times that of normal alveolar concentration. Basically the same conclusion can be drawn concerning catalytic alcohol sensors concerning the measurement accuracy of $C_{extEtOH}$, although their accuracy and reliability is still the subject of some controversy.

The variability of $C_{alvCO2}$ has been extensively studied, as evident from the physiological literature. It is generally known that the composition of alveolar gas is remarkably constant under resting conditions. This is largely because the respiratory centre of the brain is sensitive to small changes in the carbon dioxide level of arterial blood. (P.C. Johnson: The Dynamics of Respiratory Structures, in E. E. Selkurt (Ed.) Physiology 2nd Ed., Little, Brown &Co, Boston, USA, 1966, p449-450). Another stabilising factor is that a major part of the carbon dioxide within the blood volume is carried in the form of bicarbonate ions, and thus only a small fraction is exchangeable via the lungs.

It is stated that the most important factor in the control of ventilation under normal conditions is the $P_{CO2}$ of the arterial blood. The sensitivity of this control is remarkable. In the course of daily activity with periods of rests and exercise, the arterial $P_{CO2}$ is probably held to within 7% of its normal value of approximately 5 kPa (J. B. West: Respiratory Physiology - the essentials." 3rd Ed. Williams&Wilkins, Baltimore, USA, 1985, p122). It should be noted that $CO_2$ con-centration expressed as partial pressure and in unit kPa is approximately equal to that expressed as volume percent, due to the fact that normal atmospheric pressure is approximately 100 kPa.

In a study of alveolar $CO_2$ concentration in 104 human test subjects, it was found that age and gender presented weak but significant variations. The average value for women was 4.8 kPa, whereas it was 4.9 kPa for men. The age group <30 years exhibited an average of 5.0 kPa, while the group >60 years of age had an average of 4.6 kPa. Differences were also noted with respect to personality indices (A. Dhokalia, D. J. Parsons, D. E. Anderson: "Resting end-tidal CO2 association with age, gender, and personality", Psychosomatic Medicine 60 (1998) 33-37).

The alveolar $CO_2$ concentration for a human subject is known to increase somewhat with increasing physical load until a maximum is reached. At higher load, the alveolar $CO_2$ concentration deceases. The maximum value is reported to be approximately 12% higher than the resting value (M. Folke "Measurements of respiratory carbon dioxide", Department of Computer Science and Electronics, Malardalen University Doctoral Thesis No. 15, 2005, p. V2).

It has been found that if a subject hyperventilates, $C_{alvCO2}$ will temporarily decrease, and to a minor extent the opposite is possible when the subject holds his or her breath. Such variations may be minimised by repeating the determination with a time interval of a few minutes, preferably beyond the control of the subject, and taking the average of two or several determinations.

The physiological response to ambient environmental factors, such as temperature, humidity, and barometric pressure can principally be taken into account by monitoring these variables, and including their effect as correction factors in the algorithm.

Estimation of a subject's alveolar and arterial $CO_2$ concentration is thus possible, with or without taking the variations of age, gender and physical workload into account. Measurement of physical workload can be performed indirectly by measuring the subject's heart rate. From the quoted literature it is clear that the remaining error of the estimation will be of the order of $\pm 2\%$ to $\pm 10\%$ depending on whether compensation for age, gender and workload has been made, or not.

The requirements of total accuracy may vary considerably from one application to another. Highest accuracy demands are, of course, valid if the determined concentration is part of a legal process. In screening applications, the requirements may be considerably more relaxed.

From the line of reasoning, it is clear that the described method is applicable not only for the determination of alcohol concentration, but can be used for any volatile constituent within the blood circulation of a subject.

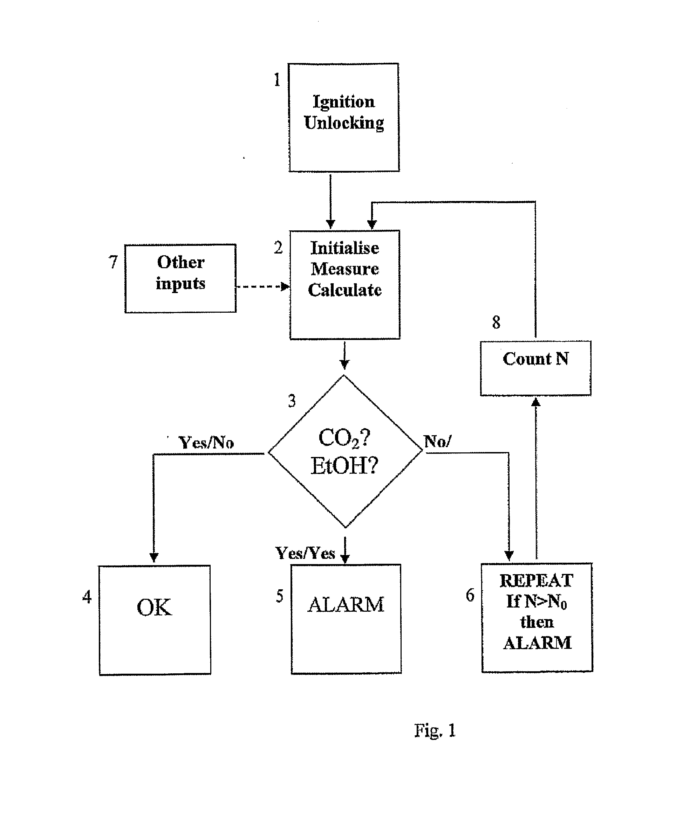

One embodiment of the method according to the invention is represented by the flow chart of Figure 1. Figure 1 illustrates to the function and almost fully automatic procedure of a device provided in a motor vehicle, with the purpose of alarming when the alcohol concentration of the expired air of the driver exceeds a certain threshold. The device is activated by turning on the ignition lock 1 of the vehicle, which is normally done manually by the driver. Alternatively, the activation can be performed automatically by means of a detector of driver presence, such as, for example, a weight sensor provided in the squab of the driver's seat.

After initial activation a sequence 2 is conducted, including an initialisation step in which the ambient air in the region of a sensor means is sampled and a measurement taken of the output signals from sensor elements, the output of which corresponds to the concentration of carbon dioxide ($CO_2$) and alcohol (ethanol, EtOH) measured at the device location. The concentration of $CO_2$ is approximately 0.04 kPa in fresh air, and could rise by 0.03-0.05 kPa due to pollution. The concentration of alcohol or other combustible gases in air is normally below 1 part per million (ppm).

The fact that the background concentrations of both gases are close to zero is employed in the present invention for automatic offset adjustment of the sensor signals. The signals obtained from the initialisation step will correspond to the background concentration, and are stored in a memory and subtracted from all subsequent signals, which will then be referred to that signal level. This procedure will thus minimise eventual offset errors from the sensor elements.

After initialisation, which may only take one or a few seconds, the driver is instructed to expire in the direction of the sensor for a few seconds. The location of the sensor means is such that it is effortlessly accessible for expiration by the driver only, at a distance of 10-50 cm. In a car, suitable locations are at the steering wheel or in the region of the top of the wind shield. The instruction for the driver to breathe against the sensor could be provided as a visual indication by e g light emitting diodes, or a sound signal, or both.

A normal exhaled breath from the driver will result in a rapidly increasing $CO_2$ concentration to a level of approximately 0.5-5 kPa, depending on the degree of dilution of the expired air in the ambient air within the cabin of the vehicle. It will then slowly return to the background level at a rate depending on the momentary airflow, diffusion etc at the device location. The degree of dilution will also depend on these factors, as well as the position of the driver, his/her ability to make the expired air hit the device, etc. From the measurements, the peak value, or an integrated or average $CO_2$ concentration over certain time is calculated for input use of $C_{extCO2}$ in the algorithm (3).

A number of criteria can be used to signal the occurrence of an 'approved' expiration (i.e. an expiration suitable for an accurate calculation of alcohol concentration), one of the simplest being a signal threshold of $C_{extCO2}$. The existence of a defined maximum concentration, or plateau is another simple criterion. Others can be related to the timing of events, and the non-existence of various types of interference, e g sharp signal 'spikes' related to electromagnetic interference that may be present in a vehicle. More or less sophisticated techniques of pattern recognition can be used for this purpose.

A simultaneous recording of the signal corresponding to alcohol concentration in the expired air is also made. A peak matching that of $CO_2$ concentration indicates that alcohol is present in the driver's expired air. The peak or time integrated value is calculated. Using the algorithm (3) with an estimated or indirectly determined value of the alveolar $CO_2$ concentration, the blood alcohol concentration is computed. The device may also be equipped with other signal inputs 7, e g the heart rate of the driver derived from electrocardiography (ECG) signals, in order to determine the driver's physical activity level.

The flow chart of Figure 1 also includes a logic step 3, categorising the signal pattern into three basic outcomes. If the expired breath is 'approved' in terms of $CO_2$ peak level, duration, etc, without the determined blood alcohol concentration exceeding a stipulated value, then the device will display an 'OK' signal 4.

If, on the other hand, the $CO_2$ signal and the determined blood alcohol concentration both exceed certain threshold values, the device will signal an of time. At the starting point 11 of the diagram, the device is switched on by alarm 5, or may even activate a disabling device disabling the ignition circuit of the vehicle to prevent the driver from operating the vehicle.

A necessary condition for the method according to the invention is that the sensor elements for $CO_2$ and alcohol are positioned so that the dilution factors of eq. (1) and (2) can be considered equal. This may be accomplished either by substantially coincident positions of the sensing elements, or by arranging connections between the $CO_2$ and alcohol sensing elements and a common sampling point. Such a connection can take the form of respective tubing combined with active or passive means for gas transportation provided within the tubing.

If the signals from neither the $CO_2$ nor the alcohol sensor elements have exceeded certain thresholds after some time after initialisation, the device will signal an indeterminate condition 6, instructing the subject to repeat the expiration against the sensor elements. The device is thus returned to the initialising and measuring phase 2 via a counter 8 which indicates the number N or time lapse of unsuccessful attempts. If a certain number of attempts No=1-5 or time is exceeded, a separate alarm indication is activated.

The method of the invention can, of course, also be used in a measuring device, rather than simply an alarm device. In a measuring device, the logic step 3 may either be simplified or completely omitted, depending on the purpose and the specific application of the device. It is also possible to combine the device with other types of devices, or use it as an embedded function of a larger system.

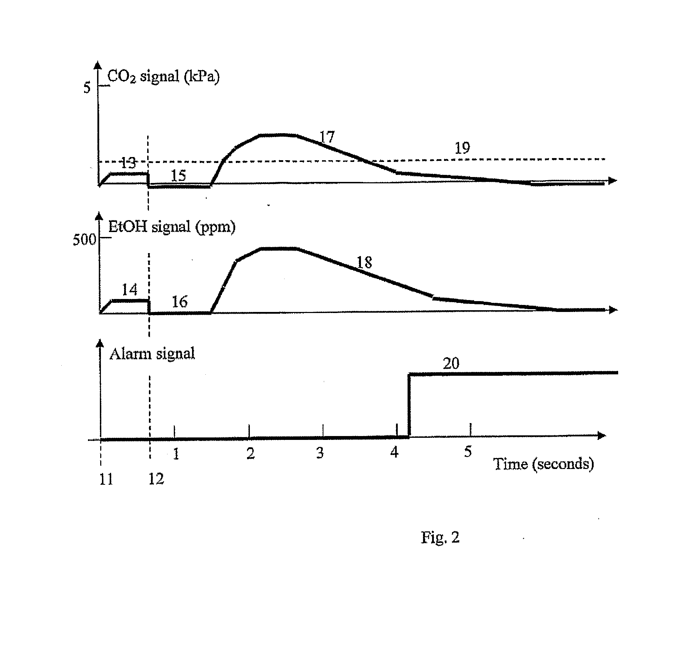

Figure 2 shows a typical time diagram of the signals used for the determination of blood alcohol concentration in the flow graph of Figure 1. In the diagram of Figure 2, the time evolution of the $CO_2$ concentration signal, the alcohol concentration signal, and the alarm signal, is displayed as a function turning the ignition key, as previously described in relation to Figure 1. The $CO_2$ and alcohol signals relatively quickly obtain a stable value corresponding to the background level of ambient air within the vehicle cabin. At point 12, offset correction is performed by subtracting the background level from the actual signal output, whereby the resulting signal goes to zero, indicated by the curve portions 15 and 16 in the $CO_2$ and alcohol signals, respectively.

Curve portion 17 indicates the $CO_2$ signal output from a single exhaled breath of the driver. The signal rapidly rises from zero to a maximum of approximately 2 kPa, indicating a degree of dilution of 2-2.5 compared to alveolar concentration. After reaching the maximum level, the signal declines, and returns to approximately zero after about five seconds.

The curve portion 18 indicates the corresponding output from the alcohol sensor element, and provides clear evidence that alcohol is present in the expired air of the driver. A comparison between the time-variation of the signal from the alcohol sensor element and the time-variation of the signal from the $CO_2$ sensor element can be performed by matching of curve portions 17 and 18 which can be the subject of more or less sophisticated pattern recognition techniques in order to establish coincidence. Such pattern recognition techniques involve a number of signatures which are compared for the two signals. Examples of signatures could be the number, time of occurrence and magnitude of peaks, or troughs. It may also be determined that the second signal is representative of the dilution of the exhaled air if the first and second signals do not contain any spikes, or other signal disturbances with signal properties and the time of frequency domain which fall outside that which can be expected from air exhaled by a subject, above a predetermined magnitude. Additionally or alternatively, the second signal may be required to exceed a predetermined threshold, or the sensed values of the first and second signals may be required to be plateau-shaped around their respective maximum values. The sampling frequency may also be required to be greater than 4Hz for the second signal to be represented of the dilution of the exhaled air. Preferably, the variation in the first and second signals is compared over at least one cycle of breath, and one cycle of breath may be considered to start when the second signal exceeds a first threshold and end when the second signal falls below a second threshold.

If the alcohol peak of curve 18 precedes that of $CO_2$ in curve 17, it is a strong indication that there is alcohol present in the upper airways of the subject, due to the simple fact that the initial expiration phase emanates from the upper airways, not visible on the $CO_2$ curve, since $CO_2$ cannot accumulate in the upper airways and must originate from the subject's lungs. A determination with alcohol present in the upper airways may not be fully representative for the blood concentration and so an alarm could be sounded or made visually. It may be recommended to repeat the measurement a few minutes after rinsing the mouth with water.

After performing signal analysis of the curve portions 17 and 18, and performing the calculations based on the algorithm (3), a logical decision can be made according to the description related to Figure 1, resulting in this case in a positive alarm signal 20 occurring soon after the occurrence of peak values of 17 and 18.

Depending on the degree of accuracy required in a given application, the algorithm (3) may or may not include correction factors to compensate for known dependence on the subject's physical activity level, e g monitored by means of his/her heart rate. Other correction factors may include dependence of environmental factors, such as temperature, humidity and barometric pressure, as discussed above.

The $CO_2$ signal is compared to a threshold value 19. If the signal does not reach this value, an accurate estimation of alcohol concentration cannot be made, and the subject will be instructed to repeat the expiration against the sensor elements. If the maximum allowed dilution of expired air is a factor 10, the threshold will be 0.5 kPa.

It should be noted that all the steps included in the method according to the invention are preferably performed completely automatically by apparatus built for the purpose. In the following description, the various elements of such apparatus will be described in some detail.

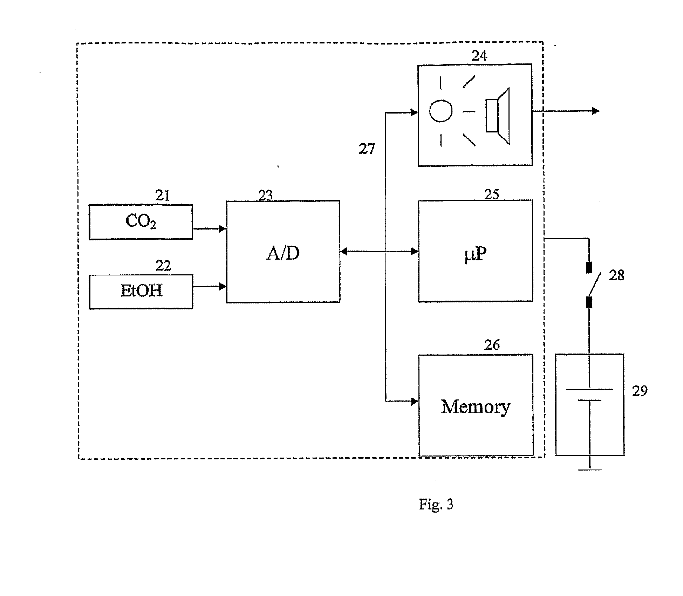

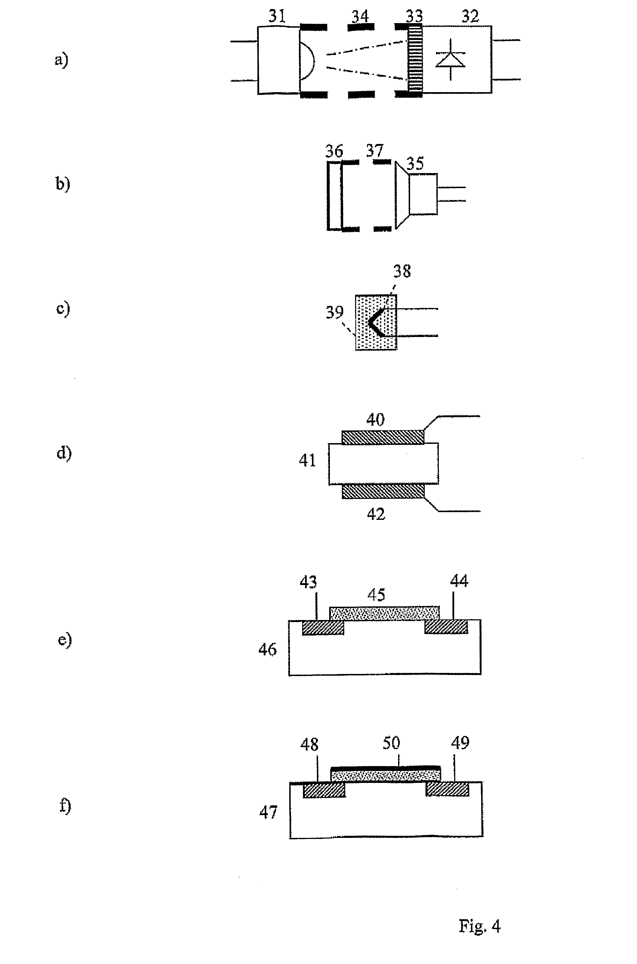

Figure 3 shows a schematic block diagram representative of one implementation of the apparatus according to the invention. As already pointed out, the apparatus includes sensor elements 21 and 22 for $CO_2$ and alcohol, respectively. The range of choices between different types of sensor elements will be described further in relation to Figure 4. In the block diagram of Figure 3, the elements 21 and 22 provide analog output voltages corresponding to their respective signal variable. These signals are converted into digital format by an analog-digital converter 23 which is communicating directly on a digital bus line 27 connecting a microprocessor 25 with a memory device 26 and a peripheral unit 24.

The microprocessor 25 includes an arithmetic-logical unit, a random access memory, and digital control circuitry, enabling relatively complex sequential operations to be performed according to a program stored in the memory 26 for permanent information storage and retrieval. The microprocessor 25 also includes a clock oscillator with precisely controlled frequency, allowing precise timing of events. Connection of an internal or external power supply 29 is performed by means of a switch 28 which could be identical to, or connected to the ignition circuit of the motor vehicle or a driver presence detector of a vehicle as described above.

The peripheral unit 24 may be an alphanumerical or graphical signal display, and may also be configured to emit audible alarm signals.

The various steps and operations described in relation to Figure 1 and 2 are contained in a sequential program stored in the memory 26, and executed upon activation of the switch 28. The program will control the microprocessor to perform all sequential operations, including the execution of the algorithm (3). It will also handle drive routines to the peripheral unit 24, and communication with other units or subsystems. The alarm output of the unit 24 may be fed back to the switch 28 (in the ignition circuit), disabling the vehicle in case of an alarm signal.

The apparatus according to the invention may also include other input elements or sensors in order to incorporate correction factors to the algorithm (3) as already discussed above. Such input elements may include a pulse sensor or electrocardiography (ECG) devices to enable the recording of the subject's heart rate, which is closely related to his/her physical activity level. Other input elements may be sensors for measuring ambient temperature, humidity and barometric pressure, enabling the algorithm (3) to be corrected for the influence of these variables.

Most of the elements of Figure 3 are made from components which are already being produced in high volume. Their cost is therefore already low, and can be further reduced by integration. Instead of employing a general purpose microprocessor, it is possible to use a dedicated field programmable gate array (FPGA), or designing one or several application specific integrated cicuits (ASICs), whereby the number of components, and hence the cost, can be further reduced.

The requirements on the sensor elements 21 and 22 are partly general and partly specific to the application, and may therefore call for specific solutions. It is required that the elements have adequate resolution, linearity, response time, stability, and immunity to other influences such as temperature, humidity, pressure and flow.

The requirement of immunity against spurious inputs can generally be solved by introducing additional sensing elements to the variables in question. Low cost sensing elements for temperature, humidity, pressure and flow are commercially available. The standard compensation technique is a simple differential arrangement between the output signals of the gas sensing element and the compensating element. Such arrangement is generally applicable, although it adds to the cost and complexity of the system.

In figure 4a)-f), six alternative gas sensing elements are schematically depicted. They all exhibit various properties of relevance to the present application. In the first two examples, 4a) and b), an inherent physical property of the gas is being measured, whereas the remaining examples rely on catalysis. It should be observed that the catalytic devices will be useful for alcohol detection only, since $CO_2$ is not combustible.

Figure 4 a) shows an optical gas sensing element, the basic function of which is to measure variations in optical transmission within a cylindrical enclosure 34, which is permeable to air or other gases. The sensing element includes a light source 31, a detector 32, and an optical bandpass filter 33 defining the wavelength region, which will differ from one gas to another, but will generally be located within the infrared spectrum range. $CO_2$ and alcohol have partly separated absorption bands at 4.3 and $3.5\mu$m, respectively. Therefore, it is basically possible to use a common light source 31 and enclosure 34 but separate detectors 32 and filters 33 for $CO_2$ and alcohol. The light source is commonly a black body radiator built from a small tungsten filament, and the detector is typically a thermopile based on the Seebeck effect, or a pyroelectric device.

To obtain high resolution at low gas concentrations, it is necessary for the optical path between the light source 31 and the detector 32 to have a length of the order of hundreds of millimetres, which may still be attainable within a small enclosure using multiple reflections. It is, however, conflicting with the requirement of short response time. Other complications with state of the art infrared solutions are that they require fine-tuning of the wavelength bands, and compensation for ageing effects.

Figure 4 b) shows an electro-acoustic gas sensing element including a two-terminal sound source 35, and an acoustic resonator built up from a permeable enclosure 37 and a sound reflecting wall 36. The sound source 35 is typically a piezoelectric device dimensioned for efficient acoustic coupling from membrane vibrations into air-borne acoustic waves. The two-terminal device will exhibit a resonance frequency determined by the average molecular mass of the gas. The presence of heavy gases, like $CO_2$ and alcohol, will lead to a decreasing resonance frequency. The arrangement has the virtue of simplicity and possibility to obtain high resolution and fast response simultaneously. However, in the present application, the response to alcohol will be completely masked by that of $CO_2$.

The sensing element shown in Fig 4 c) may be considered generic for many possible types of catalytic gas sensing devices. A heating element 38 is thus included in most catalytic devices, since the combustion of alcohol does not appear spontaneously at normal temperatures. The heating element 38 is typically a two-terminal resistive device element. Also included is the catalyst material 39 which could be tin oxide, platinum or other noble metals. Such inorganic catalysts are not specific to any particular reaction, but promote combustion processes generally. In principle, it is also possible to make use of organic catalysts, such as enzymes, which could be highly selective, and which could also operate at much lower temperatures than the inorganic catalysts. On the other hand, enzymes require an aqueous environment, and are thus poorly compatible with typical vehicle requirements.

The signal readout from the element of Fig 4 c) is based on temperature dependence of resistance. In the presence of a combustible gas, additional heat will be generated, which will alter the resistance of the element, and thus give rise to a signal. It is, of course, possible also to physically separate the resistive heating element from the heat detecting element.

A fuel cell driven by catalytic alcohol combustion is another type of alcohol sensing element, schematically depicted in Figure 4 d). A solid state electrolyte 41, is being used for ion transport between an anode 40 and a cathode 42. The combustion generates a change of the current voltage characteristics between the anode and the cathode which can be attributed to the combustion process, and hence to the presence and concentration of alcohol present.

In Figure 4 e) another catalytic device is depicted, which makes use of the resistivity variations occurring in a conductive polymer 45. The polymer is deposited on an isolating substrate 46, and the resistance is measured between two electrodes 43, 44, also deposited on the substrate.

Figure 4 f) shows a fourth example of catalytic gas sensing element based on a metal oxide semiconductor field effect transistor (MOSFET), in which the gate 50 includes a catalytic metal, e g platinum or palladium, or other noble metal. The transistor is built from a silicon substrate 47 with source 48 and drain 49 connections in addition to the gate electrode 50. Specific sensitivities to certain gases can be obtained by using an array of several such elements with different gate metal combinations ("electronic nose").

All three arrangements shown in Figure 4 d) - f) require heating elements which are not shown in the figure. Also not shown are the standard preamplifiers required to provide a signal output voltage for the analog/digital converter 23 of Figure 3.

The arrangements shown in Figure 4 a)-f) may be miniaturised and produced at very low cost using micro electro mechanical systems (MEMS) technology. This technology allows batch fabrication by micromachining, whereby relatively complex structures are determined by photolithography, and a combination of additive deposition or bonding, and subtractive etching. The physical dimensions of the entire system of Figure 3 may be 50 x 50 x 20 mm or less, and the unit cost could be very low in high production volume.

When used in this specification and claims, the terms "comprises" and "comprising" and variations thereof mean that the specified features, steps or integers are included. The terms are not to be interpreted to exclude the presence of other features, steps or components.

## Claims

1. A method of assessing a blood concentration level, in a human or animal subject, of ethanol, the method comprising the steps of:

    positioning a sensor means (21,22) within an expiratory gas-flow of the subject, the sensor means (21,22) being configured to detect the presence of ethanol and provide a first output signal ($C_{extEtOH}$) representative of the concentration of the ethanol in air, and to detect the presence of carbon dioxide and provide a second output signal ($C_{ext}CO_2$) representative of the concentration of carbon dioxide in air;
    sampling a flow of expiratory gases from the subject using the sensor (21,22) to provide the first output signal ($C_{extEtOH}$) and the second output signal ($C_{ext}CO_2$) for the expiratory gases simultaneously; :
    comparing the variation of the first output signal ($C_{extEtOH}$) for the expiratory gases over time with the variation of the second output signal ($C_{ext}CO_2$) for the expiratory gases over time by plotting the time-variation of the first output signal ($C_{extEtOH}$) and the second output signal ($C_{ext}CO_2$) and matching a curve portion of the first output signal ($C_{extEtOH}$) with a curve portion the second output signal ($C_{ext}CO_2$) and, depending on the result of the comparison if a curve portion of the first output signal ($C_{extEtOH}$) matches a curve portion of the second output signal ($C_{ext}CO_2$):
    making the second output signal ($C_{ext}CO_2$) for the expiratory gases representative of a degree of dilution of the expiratory airflow to adjust the concentration of ethanol represented by the first output signal ($C_{extEtOH}$);
    sampling ambient air and using the sensor means (21,22) to provide the first and second output signals representative of ethanol concentration ($C_{extEtOH}$) and carbon-dioxide concentration ($C_{ext}CO_2$) in the ambient air at the location of the sensor means (21,22);
    correcting the first and second output signals for the expiratory gases by subtracting the first and second output signals representative of ambient air from the first and second output signals for the expiratory gases to yield corrected first and second output signals ($C_{extEtOH}$, $C_{ext}CO_2$) for the expiratory gases,
dividing the corrected first signal ($C_{extEtOH}$) representative of the concentration of ethanol in the expiratory gases by the corrected second signal ($C_{ext}CO_2$) representative of the concentration of carbon dioxide in the expiratory gases to yield a ratio ($C_{extEtOH}/C_{ext}CO_2$);
multiplying the ratio ($C_{extEtOH}/C_{ext}CO_2$) by a factor representative of the subject's alveolar carbon dioxide concentration ($C_{alv}CO_2$) to define the adjusted concentration of ethanol ($C_{alvEtOH}$); and
determining the blood concentration level of ethanol wherein the degree of dilution is defined by the concentration of carbon dioxide in the expiratory gases divided by the subject's alveolar carbon dioxide concentration defining ($C_{ext}CO_2$)/($C_{alv}CO_2$).

2.  A method according to claim 1, including the step of estimating said factor on the basis of one or more subject-specific parameters selected from the group comprising age, gender, weight, personality index and fitness index.

3.  A method according to claim 2, including the step of measuring the subject's heart rate to calculate said fitness index.

4.  A method according to any preceding claim, wherein the steps of sampling and inputting are repeated at a repetition rate beyond the control of the subject.

5.  A method according to any preceding claim, wherein said sensor means comprises an ethanol sensor and a carbon dioxide-sensor, the sensor means being configured such that both sensors sample gas at substantially the same location in said expiratory gas-flow.

6.  A method according to any preceding claim, wherein said step of positioning involves instructing the subject to expire towards the sensor means from a maximum predetermined range.

7.  A method according to claim 6, wherein said range is approximately 0.5 metres.

8.  A method according to claim 6 or 7, wherein said instruction is given by way of visual and/or audible signals.

9.  A method according to any preceding claim, wherein said second output signal in respect of said expiratory gases is the peak value of carbon dioxide concentration detected during said sampling step.

10.  A method according to any one of claims 1 to 8, wherein said second output signal in respect of said expiratory gases is an integrated or average value of carbon dioxide concentration detected during said sampling step.

11.  A method according to any preceding claim, wherein said first output signal in respect of said expiratory gases is the peak value of ethanol-concentration detected during said sampling step.

12.  A method according to any preceding claim, wherein the subject is the driver of a motor vehicle and the method is conducted within the vehicle.

13.  A method according to any preceding claim, wherein the variation of the first and second output signals over time is compared over a time period of at least one cycle of breath.

14.  A method according to claim 13, wherein a cycle of breath is determined to start when the second output signal exceeds the first threshold and to end when the second output signal falls below a second threshold.

15.  A method according to any preceding claim, wherein the second output signal is determined to be representative of the degree of dilution of the expiratory airflow if the first and second output signals do not contain spikes or other signal disturbances having properties in the time or frequency domain which fall outside that which can be expected from breath exhaled by subject, above a predetermined magnitude.

16.  A method according to any preceding claim, wherein the second output signal is determined to be representative of the degree of dilution of the expiratory airflow if the second signal exceeds a predetermined threshold.

17.  A method according to any preceding claim, wherein the second output signal is determined to be representative of the degree of dilution to the expiratory airflow if the first and second output signals are substantially plateau-shaped around their respective maximum values.

18. A method according to any preceding claim, wherein the frequency of sampling the first and second output signals is equal to or greater than around 4 Hz.

19. An apparatus configured to implement the method of any preceding claim, the apparatus comprising a sensor means (21,22) positionable within the expiratory gas-flow of the subject, the sensor means (21,22) being configured to detect the presence of ethanol and provide the first output signal representative of the concentration of the ethanol in air, and to detect the presence of carbon dioxide and provide a second output signal representative of the concentration of carbon dioxide in air; a memory (26) carrying an algorithm; and a processor (25) configured to process said signals in accordance with the algorithm stored in the memory (26).

20. An apparatus according to claim 19 provided in a motor vehicle.

21. An apparatus according to claim 20, wherein said sensor means comprises at least one sensor element (21,22) mounted within said motor vehicle at a position in front of the driver's seat of the vehicle.

22. An apparatus according to claim 22 or 23, wherein said processor is operatively connected to a switch (28) in the ignition circuit of the vehicle, the processor (25) being configured to close the switch (28) if the result of the algorithm is below a predetermined blood concentration value of ethanol.

## Patentansprüche

1. Verfahren zum Schätzen einer Blutkonzentration von Ethanol in einem menschlichen oder tierischen Lebewesen, wobei das Verfahren folgende Schritte umfasst:

Platzieren eines Sensormittels (21, 22) innerhalb eines Ausatemgasstroms des Lebewesens, wobei das Sensormittel (21, 22) so ausgelegt ist, dass es das Vorhandensein von Ethanol erfasst und ein erstes Ausgangssignal ($C_{extEtOH}$) liefert, das die Konzentration von Ethanol in Luft darstellt, und das Vorhandensein von Kohlendioxid erfasst und ein zweites Ausgangssignal ($C_{ext}CO_2$) liefert, das die Kohlendioxidkonzentration in Luft darstellt;

Probenahme aus einem Strom von Ausatemgasen von dem Lebewesen unter Verwendung des Sensors (21, 22) zum gleichzeitigen Liefern des ersten Ausgangssignals ($C_{extEtOH}$) und des zweiten Ausgangssignals ($C_{ext}CO_2$) für die Ausatemgase;

Vergleichen der zeitlichen Veränderung des ersten Ausgangssignals ($C_{ex-tEtOH}$) für die Ausatemgase mit der zeitlichen Veränderung des zweiten Ausgangssignals ($C_{ext}CO_2$) für die Ausatemgase durch Auftragen der zeitlichen Veränderung des ersten Ausgangssignals ($C_{extEtOH}$) und des zweiten Ausgangssignals ($C_{ext}CO_2$) und Vergleichen eines Kurvenabschnitts des ersten Ausgangssignals ($C_{extEtOH}$) mit einem Kurvenabschnitt des zweiten Ausgangssignals ($C_{ext}CO_2$) und, je nach dem Ergebnis des Vergleichs, wenn ein Kurvenabschnitt des ersten Ausgangssignals ($C_{extEtOH}$) einem Kurvenabschnitt des zweiten Ausgangssignals ($C_{ext}CO_2$) entspricht:

Veranlassen, dass das zweite Ausgangssignal ($C_{ext}CO_2$) für die Ausatemgase einen Verdünnungsgrad des Ausatemluftstroms darstellt, damit die Ethanolkonzentration angepasst wird, die von dem ersten Ausgangssignal ($C_{extEtOH}$) dargestellt wird;

Probenahme aus Umgebungsluft und Verwenden des Sensormittels (21, 22) zum Liefern des ersten und zweiten Ausgangssignals, das die Ethanolkonzentration ($C_{extEtOH}$) und Kohlendioxidkonzentration ($C_{ext}CO_2$) in der Umgebungsluft an dem Ort des Sensormittels (21, 22) darstellt;

Korrigieren des ersten und zweiten Ausgangssignals für die Ausatemgase durch Abziehen des ersten und zweiten Ausgangssignals, die Umgebungsluft darstellen, von dem ersten und zweiten Ausgangssignal für die Ausatemgase, damit sich ein korrigiertes erstes und zweites Ausgangssignal ($C_{extEtOH}$, $C_{ext}CO_2$) für die Ausatemgase ergibt,

Teilen des korrigierten ersten Signals ($C_{extEtOH}$), das die Konzentration von Ethanol in den Ausatemgasen darstellt, durch das korrigierte zweite Signal ($C_{ext-}CO_2$), das die Kohlendioxidkonzentration in den Ausatemgasen darstellt, damit sich ein Verhältnis ($C_{extEtOH}/C_{ext}CO_2$) ergibt;

Multiplizieren des Verhältnisses ($C_{extEtOH}/C_{ext}CO_2$) mit einem Faktor, der die Kohlendioxidkonzentration in den Lungenbläschen ($C_{alv}CO_2$) des Lebewesens darstellt, damit die angepasste Ethanolkonzentration ($C_{alvEtOH}$) definiert wird; und

Ermitteln der Blutkonzentration von Ethanol, wobei der Verdünnungsgrad durch die Kohlendioxidkonzentration in den Ausatemgasen, geteilt durch die Kohlendioxidkonzentration in den Lungenbläschen des Lebewesens definiert ist, womit ($C_{ext}CO_2$)/($C_{alv}CO_2$) definiert ist.

2. Verfahren nach Anspruch 1, das den Schritt des Abschätzens des Faktors auf der Grundlage von einem oder mehreren lebewesenspezifischen Parametern umfasst, die aus der Gruppe umfassend Alter, Geschlecht, Gewicht, Persönlichkeitsindex und Fitnessindex ausgewählt werden.

3. Verfahren nach Anspruch 2, das den Schritt des Messens der Herzfrequenz des Lebewesens zum Berechnen des Fitnessindex umfasst.

4. Verfahren nach einem vorhergehenden Anspruch, wobei die Schritte der Probenahme und des Eingebens mit einer Wiederholfrequenz wiederholt werden, die außerhalb des Einflussbereichs des Lebewesens liegt.

5. Verfahren nach einem hervorgehenden Anspruch, wobei das Sensormittel einen Ethanolsensor und einen Kohlendioxidsensor umfasst, wobei das Sensormittel so ausgelegt ist, dass beide Sensoren an im Wesentlichen demselben Ort in dem Ausatemgasstrom eine Gasprobenahme durchführen.

6. Verfahren nach einem vorhergehenden Anspruch, wobei der Schritt des Platzierens umfasst, dass das Lebewesen angewiesen wird, aus einer festgelegten maximalen Entfernung in Richtung des Sensormittels auszuatmen.

7. Verfahren nach Anspruch 6, wobei die Entfernung ungefähr 0,5 Meter beträgt.

8. Verfahren nach Anspruch 6 oder 7, wobei die Anweisung mittels optischen und/oder akustischen Signalen gegeben wird.

9. Verfahren nach einem vorhergehenden Anspruch, wobei das zweite Ausgangssignal bezogen auf die Ausatemgase der Spitzenwert der während des Probenahmeschritts erfassten Kohlendioxidkonzentration ist.

10. Verfahren nach einem der Ansprüche 1 bis 8, wobei das zweite Ausgangssignal bezogen auf die Ausatemgase ein integrierter oder durchschnittlicher Wert der während des Probenahmeschritts erfassten Kohlendioxidkonzentration ist.

11. Verfahren nach einem vorhergehenden Anspruch, wobei das erste Ausgangssignal bezogen auf die Ausatemgase der Spitzenwert der während des Probenahmeschritts erfassten Ethanolkonzentration ist.

12. Verfahren nach einem vorhergehenden Anspruch, wobei das Lebewesen der Fahrer eines Kraftfahrzeugs ist und das Verfahren innerhalb des Fahrzeugs durchgeführt wird.

13. Verfahren nach einem vorhergehenden Anspruch, wobei die zeitliche Veränderung des ersten und zweiten Ausgangssignals über einen Zeitraum von mindestens einem Atemzyklus verglichen wird.

14. Verfahren nach Anspruch 13, wobei festgelegt wird, dass ein Atemzyklus beginnt, wenn das zweite Ausgangssignal die erste Schwelle übersteigt, und endet, wenn das zweite Ausgangssignal unter eine zweite Schwelle fällt.

15. Verfahren nach einem vorhergehenden Anspruch, wobei festgelegt wird, dass das zweite Ausgangssignal den Verdünnungsgrad des Ausatemluftstroms darstellt, wenn das erste und zweite Ausgangssignal keine Nadelimpulse oder anderen Signalstörungen mit Eigenschaften in der Zeit- oder Frequenzdomäne enthält, die außerhalb dessen liegen, was von Atem zu erwarten ist, den ein Lebewesen ausatmet, über einer vorher festgelegten Höhe.

16. Verfahren nach einem vorhergehenden Anspruch, wobei festgelegt wird, dass das zweite Ausgangssignal den Verdünnungsgrad des Ausatemluftstroms darstellt, wenn das zweite Signal eine vorher festgelegte Schwelle übersteigt.

17. Verfahren nach einem vorhergehenden Anspruch, wobei festgelegt wird, dass das zweite Ausgangssignal den Verdünnungsgrad zum Ausatemluftstrom darstellt, wenn das erste und zweite Ausgangssignal um ihre jeweiligen Höchstwerte herum im Wesentlichen plateauförmig sind.

18. Verfahren nach einem vorhergehenden Anspruch, wobei die Frequenz der Abtastung des ersten und zweiten Ausgangssignals ungefähr 4 Hz entspricht oder darüber liegt.

19. Vorrichtung, die so ausgelegt ist, dass sie das Verfahren nach einem vorhergehenden Anspruch ausführt, wobei die Vorrichtung ein Sensormittel (21, 22) umfasst, das innerhalb des Ausatemgasstroms des Lebewesens platzierbar ist, wobei das Sensormittel (21, 22) so ausgelegt ist, dass es das Vorhandensein von Ethanol erfasst und das erste Ausgangssignal liefert, das die Konzentration von Ethanol in Luft darstellt, und das Vorhandensein von Kohlendioxid erfasst und ein zweites Ausgangssignal liefert, das die Kohlendioxidkonzentration in Luft darstellt; einen Speicher (26), der einen Algorithmus enthält; und einen Prozessor (25), der so ausgelegt ist, dass er die Signale gemäß dem in dem Speicher (26) gespeicherten Algorithmus verarbeitet.

**20.** Vorrichtung nach Anspruch 19, die in einem Kraftfahrzeug angeordnet ist.

**21.** Vorrichtung nach Anspruch 20, wobei das Sensormittel mindestens ein Sensorelement (21, 22) umfasst, das in dem Kraftfahrzeug an einer Stelle vor dem Fahrersitz des Fahrzeugs montiert ist.

**22.** Vorrichtung nach Anspruch 22 oder 23, wobei der Prozessor funktionsmäßig mit einem Schalter (28) in dem Zündschaltkreis des Fahrzeugs verbunden ist, wobei der Prozessor (25) so ausgelegt ist, dass er den Schalter (28) schließt, wenn das Ergebnis des Algorithmus unter einem vorher festgelegten Blutkonzentrationswert für Ethanol liegt.

**Revendications**

**1.** Procédé d'évaluation d'un niveau de concentration d'éthanol dans le sang, dans un sujet humain ou animal, le procédé comprenant les étapes consistant à :

positionner un moyen détecteur (21, 22) dans un flux de gaz expiré du sujet, le moyen détecteur (21, 22) étant configuré pour détecter la présence d'éthanol et fournir un premier signal de sortie ($C_{extEtOH}$) représentatif de la concentration de l'éthanol dans l'air, et pour détecter la présence de dioxyde de carbone et fournir un second signal de sortie ($C_{ext}CO_2$) représentatif de la concentration de dioxyde de carbone dans l'air ;

prélever un échantillon d'un flux de gaz expirés du sujet au moyen du détecteur (21, 22) afin de fournir simultanément le premier signal de sortie ($C_{extEtOH}$) et le second signal de sortie ($C_{ext}CO_2$) pour les gaz expirés ;

comparer la variation du premier signal de sortie ($C_{extEtOH}$) pour les gaz expirés dans le temps avec la variation du second signal de sortie ($C_{ext}CO_2$) pour les gaz expirés dans le temps en traçant la variation dans le temps du premier signal de sortie ($C_{extEtOH}$) et du second signal de sortie ($C_{ext}CO_2$) et en comparant une partie de courbe du premier signal de sortie ($C_{extEtOH}$) avec une partie de courbe du second signal de sortie ($C_{ext}CO_2$) et, en fonction du résultat de la comparaison, si une partie de courbe du premier signal de sortie ($C_{extEtOH}$) correspond à une partie de courbe du second signal de sortie ($C_{ext}CO_2$) :

rendre le second signal de sortie ($C_{ext}CO_2$) pour les gaz expirés représentatif d'un degré de dilution du flux d'air expiré pour ajuster la concentration d'éthanol représentée par le premier signal de sortie ($C_{extEtOH}$) ;

prélever un échantillon d'air ambiant et utiliser le moyen détecteur (21, 22) pour fournir les premier et second signaux de sortie représentatifs de la concentration d'éthanol ($C_{extEtOH}$) et de la concentration de dioxyde de carbone ($C_{ext}CO_2$) dans l'air ambiant à l'emplacement du moyen détecteur (21, 22) ;

corriger les premier et second signaux de sortie pour les gaz expirés en soustrayant les premier et second signaux de sortie représentatifs de l'air ambiant des premier et second signaux de sortie pour les gaz expirés, afin d'obtenir des premier et second signaux de sortie ($C_{extEtOH}$, $C_{ext}CO_2$) corrigés pour les gaz expirés,

diviser le premier signal ($C_{extEtOH}$) corrigé représentatif de la concentration d'éthanol dans les gaz expirés par le second signal ($C_{ext}CO_2$) corrigé représentatif de la concentration de dioxyde de carbone dans les gaz expirés afin d'obtenir un rapport ($C_{extEtOH}/C_{est}CO_2$) ;

multiplier le rapport ($C_{extEtOH}/C_{ext}CO_2$) par un facteur représentatif de la concentration de dioxyde de carbone alvéolaire ($C_{alv}CO_2$) du sujet pour définir la concentration ajustée d'éthanol ($C_{alvEtOH}$) ; et

déterminer le niveau de concentration d'éthanol dans le sang, le degré de dilution étant défini par la concentration de dioxyde de carbone dans les gaz expirés divisée par la définition de la concentration de dioxyde de carbone alvéolaire ($C_{ext}CO_2$)/($C_{alv}CO_2$) du sujet.

**2.** Procédé selon la revendication 1, comportant l'étape consistant à estimer ledit facteur sur la base d'un ou de plusieurs paramètres spécifiques du sujet, choisi(s) dans le groupe comprenant l'âge, le genre, le poids, l'indice de personnalité et l'indice de forme physique.

**3.** Procédé selon la revendication 2, comportant l'étape consistant à mesurer la fréquence cardiaque du sujet pour calculer ledit indice de forme physique.

**4.** Procédé selon l'une quelconque des revendications précédentes, dans lequel les étapes de prélèvement d'échantillon et d'entrée sont répétées à une fréquence de répétition au-delà du contrôle du sujet.

**5.** Procédé selon l'une quelconque des revendications précédentes, dans lequel ledit moyen détecteur comprend un détecteur d'éthanol et un détecteur de dioxyde de carbone, le moyen détecteur étant configuré de telle manière que les deux détecteurs prélèvent un échantillon de gaz essentiellement au même endroit dans ledit flux de gaz expiré.

**6.** Procédé selon l'une quelconque des revendications précédentes, dans lequel ladite étape de positionnement implique de donner l'instruction au sujet d'expirer vers le moyen détecteur depuis une portée maximale prédéterminée.

**7.** Procédé selon la revendication 6, dans lequel ladite portée est d'environ 0,5 mètre.

**8.** Procédé selon la revendication 6 ou 7, dans lequel ladite instruction est donnée au moyen de signaux visuels et/ou audibles.

**9.** Procédé selon l'une quelconque des revendications précédentes, dans lequel ledit second signal de sortie en ce qui concerne lesdits gaz expirés est la valeur maximale de la concentration de dioxyde de carbone détectée pendant ladite étape de prélèvement d'échantillon.

**10.** Procédé selon l'une quelconque des revendications 1 à 8, dans lequel ledit second signal de sortie en ce qui concerne lesdits gaz expirés est une valeur intégrée ou moyenne de la concentration de dioxyde de carbone détectée pendant ladite étape de prélèvement d'échantillon.

**11.** Procédé selon l'une quelconque des revendications précédentes, dans lequel ledit premier signal de sortie en ce qui concerne lesdits gaz expirés est la valeur maximale de la concentration d'éthanol détectée pendant ladite étape de prélèvement d'échantillon.

**12.** Procédé selon l'une quelconque des revendications précédentes, dans lequel le sujet est le conducteur d'un véhicule à moteur et le procédé est exécuté dans le véhicule.

**13.** Procédé selon l'une quelconque des revendications précédentes, dans lequel la variation des premier et second signaux de sortie dans le temps est comparée sur une période de temps d'au moins un cycle respiratoire.

**14.** Procédé selon la revendication 13, dans lequel un cycle respiratoire est déterminé comme commençant quand le second signal de sortie dépasse le premier seuil et se terminant quand le second signal de sortie tombe sous un second seuil.

**15.** Procédé selon l'une quelconque des revendications précédentes, dans lequel le second signal de sortie est déterminé comme étant représentatif du degré de dilution du flux d'air expiré si les premier et second signaux de sortie ne contiennent ni pic ni autre perturbation de signal présentant des propriétés dans le domaine du temps ou de la fréquence qui se trouvent hors de ce que l'on peut attendre de la respiration expirée par le sujet, au-dessus d'une grandeur prédéterminée.

**16.** Procédé selon l'une quelconque des revendications précédentes, dans lequel le second signal de sortie est déterminé comme étant représentatif du degré de dilution du flux d'air expiré si le second signal dépasse un seuil prédéterminé.

**17.** Procédé selon l'une quelconque des revendications précédentes, dans lequel le second signal de sortie est déterminé comme étant représentatif du degré de dilution par rapport au flux d'air expiré si les premier et second signaux de sortie sont essentiellement en forme de plateau autour de leur valeur maximale respective.

**18.** Procédé selon l'une quelconque des revendications précédentes, dans lequel la fréquence de prélèvement d'échantillon des premier et second signaux de sortie est égale ou supérieure à environ 4 Hz.

**19.** Appareil configuré pour mettre en oeuvre le procédé selon l'une quelconque des revendications précédentes, l'appareil comprenant un moyen détecteur (21, 22) positionnable dans le flux de gaz expiré du sujet, le moyen détecteur (21, 22) étant configuré pour détecter la présence d'éthanol et fournir le premier signal de sortie représentatif de la concentration de l'éthanol dans l'air, et pour détecter la présence de dioxyde de carbone et fournir un second signal de sortie représentatif de la concentration de dioxyde de carbone d'ans l'air ; une mémoire (26) contenant un algorithme ; et un processeur (25) configuré pour traiter lesdits signaux en fonction de l'algorithme enregistré dans la mémoire (26).

**20.** Appareil selon la revendication 19, mis à disposition dans un véhicule à moteur.

**21.** Appareil selon la revendication 20, dans lequel ledit moyen détecteur comprend au moins un élément détecteur (21, 22) monté dans ledit véhicule à moteur dans une position en face du siège du conducteur du véhicule.

**22.** Appareil selon la revendication 22 ou 23, dans lequel ledit processeur est relié de manière fonctionnelle à un interrupteur (28) dans le circuit d'allumage du véhicule, le processeur (25) étant configuré pour fermer l'interrupteur (28) si le résultat de l'algorithme est inférieur à une valeur prédéterminée de concentration d'éthanol dans le sang.

Fig. 1

Fig. 2

Fig. 3

a)

31    34    33    32

b)

36    37    35

c)

38
39

d)

40
41
42

e)

43    45    44
46

f)

48    50    49
47

Fig. 4

# REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1441212 A

**Non-patent literature cited in the description**

- The Dynamics of Respiratory Structures. **P.C. JOHNSON.** Physiology. Little, Brown &Co, 1966, 449-450
- **J. B. WEST.** Respiratory Physiology - the essentials. Williams&Wilkins, 1985, 122
- **A. DHOKALIA ; D. J. PARSONS ; D. E. ANDERSON.** Resting end-tidal CO2 association with age, gender, and personality. *Psychosomatic Medicine,* 1998, vol. 60, 33-37
- **M. FOLKE.** Measurements of respiratory carbon dioxide. *Department of Computer Science and Electronics, Malardalen University Doctoral Thesis,* 2005, V2